# EUROPEAN PATENT APPLICATION

(11) **EP 1 752 451 A1**
(43) Date of publication of application: **14.02.2007**
(21) Application number: 05740948.4
(22) Date of filing: 17.05.2005
(51) Int. Cl.: C07D 233/50, C07F 3/06

(54) **BISGUANIDINE-COMPOUND OPTICAL RESOLVER AND SEPARATING AGENT FOR CHIRAL MOLECULE**

(30) Priority: 17.05.2004 JP 2004146883
(71) Applicant: Japan Science and Technology Agency, Kawaguchi-shi, Saitama 332-0012 (JP); National University Corporation Chiba University, Chiba-shi Chiba, 2638522 (JP)
(72) Inventor: ISHIKAWA, Tsutomu, Ichihara-shi, Chiba 290-0143 (JP); KAWAHATA, Masatoshi, Chiba-shi, chiba 263-0024 (JP)
(74) Representative: Calamita, Roberto
(86) International application number: PCT/JP2005/009300
(87) International publication number: WO 2005/110997

(57) **Abstract**

A new guanidine compound which is a bisguanidine compound represented by the formula (1): (wherein X represents hydrogen or a substituent; the benzene ring may have the same or different substituents bonded thereto; and R¹'s are the same or different hydrocarbon groups) and has a symmetrical structure. This compound can be a new functional substance such as, e.g., an aid for asymmetric synthesis.

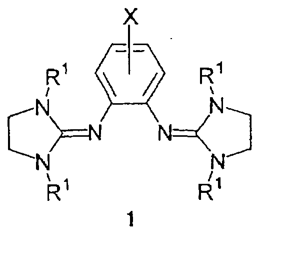

## Description

### Technical Field

The invention of this application relates to a bisguanidine compound. More specifically, it relates to a new bisguanidine compound which can be developed as a functional molecule of an optical resolver, a chiral molecule separation type, an organic base or acid substance scavenger or the like, or as diversified functional derivatives being synthetic intermediates and which is useful in the fields of asymmetric synthesis and separative purification of chemical products such as medicaments, agrochemicals, perfume and cosmetics, environmental purification and the like, a process for producing the same, and an optical resolver and the like containing the same as an active ingredient.

### Technical Background

Various chiral compounds have been so far utilized as an aid for asymmetric synthesis. However, all of these compounds are mainly compounds having chiral center such as an amino acid in the molecule or dicyclic biphenol compounds with axial asymmetry such as binaphthol. Monocyclic, axially asymmetric compounds have not been to date taken into consideration.

Meanwhile, the inventors of this application have discovered that a guanidine compound is effective as a functional reagent in various chemical synthesis reactions and this compound can be used as a solid phase reaction reagent by being immobilized on a polymer carrier, and they have so far made various proposals (Patent Documents 1 and 2). They have also proposed that the guanidine compound is developed as a chiral base compound by modification (non-Patent Document 1).

Under these circumstances, the inventors of this application have studied the development of a symmetric functional compound such as an aid for asymmetric synthesis based on the guanidine compound to replace ordinary chiral compounds.
Patent Document 1: Gazette of JP-A-2003-306482
Patent Document 2: Gazette of JP-A-2002-201175
Non-Patent Document 1: J. Org. Chem., 2000, 65, 7770-7773, 7774-7778, 7779-7785

### Disclosure of Invention

Under the foregoing circumstances, the invention of this application aims to provide a new guanidine compound having a symmetric structure and to develop this compound as a new functional substance such as an aid for asymmetric synthesis.

This application provides the following inventions to solve the foregoing problems.
[1] An optical resolver containing as an active ingredient a bisguanidine compound represented by the following formula (1) 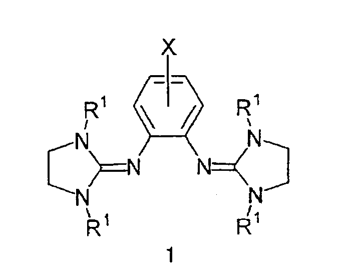 (wherein X represents a hydrogen atom or a substituent, a benzene ring may have the same or different plural substituents bonded thereto, and R¹'s represent the same or different hydrocarbon groups).
[2] A chiral molecule separating agent containing as an active ingredient a bisguanidine compound represented by the following formula (1) 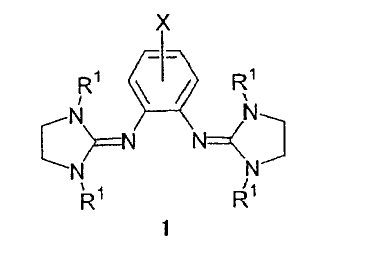 (wherein X represents a hydrogen atom or a substituent, a benzene ring may have the same or different plural substituents bonded thereto, and R¹'s represent the same or different hydrocarbon groups).
[3] A complex of a bisguanidine compound represented by the following formula (1) 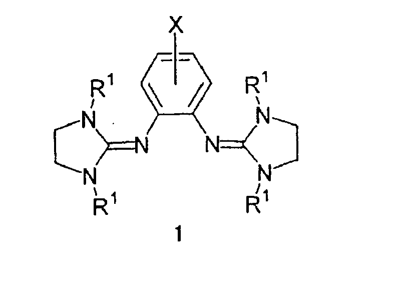 (wherein X represents a hydrogen atom or a substituent, a benzene ring may have the same or different plural substituents bonded thereto, and R¹'s represent the same or different hydrocarbon groups), and a metal.
[4] An organic base or cation scavenger containing as an active ingredient a bisguanidine compound represented by the following formula (1) 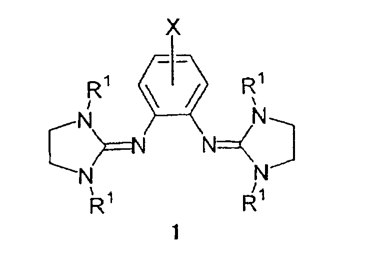 (wherein X represents a hydrogen atom or a substituent, a benzene ring may have the same or different plural substituents bonded thereto, and R¹'s represent the same or different hydrocarbon groups).
[5] A process for producing a bisguanidine compound represented by the following formula (1) 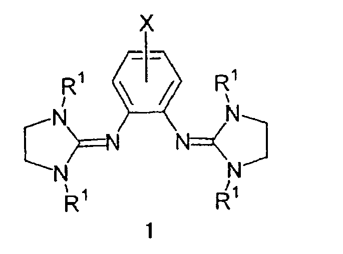 (wherein X represents a hydrogen atom or a substituent, a benzene ring may have the same or different plural substituents bonded thereto, and R¹'s represent the same or different hydrocarbon groups),
   which comprises reacting a phenylenediamine compound represented by the following formula 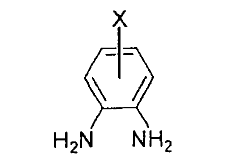 (wherein X represents a hydrogen atom or a substituent, and a benzene ring may have the same or different plural substituents bonded thereto)
   with an imidazolinium compound represented by the following formula 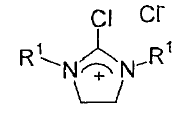 (wherein R¹'s represent the same or different hydrocarbon groups).

### Best Mode for Carrying Out the Invention

The invention of this application has the foregoing characteristic features, and the embodiments thereof are described below.

In the bisguanidine compound represented by the formula (1) in the invention of this application, symbol X is a hydrogen atom or a substituent. With respect to the substituent in this case, the same or different plural groups may be bonded to the benzene ring. When X is the substituent, it may include various groups. Examples thereof include a hydrocarbon group, as well as an aldehyde group, a ketone group, a hydroxyl group, an amino group, an alkoxy group, a carboxyl group, an alkoxycarbonyl group, a carbonyloxy group, a halogen atom and the like. Further, substituted hydrocarbon groups containing the same are also available. When X is a hydrocarbon group, it may include various groups such as 3,4-dimethyl, 3,4-diethyl, 3,4-dipropyl, 3,4-dibutyl, 2,5-dimethyl, 2,5-diethyl and 3-methyl-4-ethyl. The carbon number thereof is considered to be from 1 to 6 as a standard. Further, symbol R¹'s represent the same or different hydrocarbon groups which may have a substituent. These hydrocarbon groups may include various groups such as methyl, ethyl, propyl, butyl, isobutyl and t-butyl. The carbon number thereof is considered to be from 1 to 8 as a standard.

Such a bisguanidine compound can easily be produced by, for example, a reaction of an o-phenylenediamine compound and an imidazolinium compound according to the following formula.

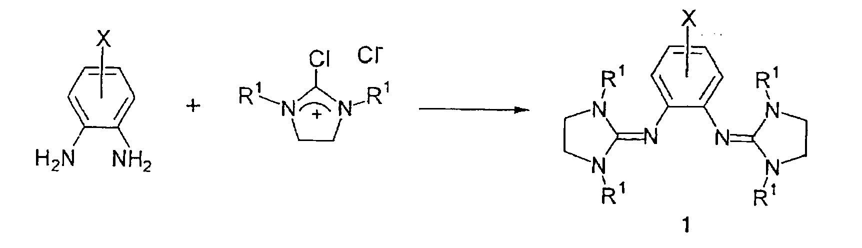

The bisguanidine compound in the invention of this application attracts much interest as a compound having functionality as an optical resolver, a chiral molecule separating agent, an organic base or acid substance scavenger or the like. For example, the inventors of this application reacted commercial o-phenylenediamine with 2-chloro-1,3-dimethylimidazolinium chloride to form a bisguanidine compound 1 (R¹=R²=Me, X=H). This compound easily formed a complex with benzoic acid, phenol and benzyl alcohol, and an expected function as an organic base was confirmed. Further, from the consideration of a molecular model, it was presumed that guanidiyl groups present adjacent to the benzene ring by restricted rotation were located above and below the plane of the benzene ring and the compound was optically resolved into (P) isomer and (M) isomer. Accordingly, the compound was formed into a salt using a benzoyltartaric acid, and X-ray crystal structure analysis was conducted. It was consequently found that (P)-bisguanidine formed a complex with (R,R) isomer and (M)-bisguanidine formed a complex with (S,S) isomer. The bisguanidine compounds are liberated from these complexes to form optically active bisguanidine compounds.

Similarly, bisguanidine compounds 1 having different substituents (for example, R¹=R²=Et, X=H; R¹=R²=Me, X=3,6-diBr) are also formed, and the same functionality is confirmed.

It is possible to supply at low cost chemical chiral products such as medicaments, agrochemicals, perfume and cosmetics by an efficient asymmetric reaction using this functional reagent, to establish a new separation system as a chiral column carrier, to purify water by selective extraction of metallic cations using polymeric derivatives, and the like.

In the invention of this application, a complex with a metal by mixing with a metallic compound or the like is also provided. A compound having functionality as a catalyst, a synthetic intermediate or the like is provided.

The invention is illustrated more specifically below by referring to Examples. Of course, the invention is not limited by the following Examples.

### Examples

### (Experiment)

Proton and carbon nuclear magnetic resonance spectra were measured with JNM-ECP400 of JEOL, Ltd. in dichloroform using tetramethylsilane as an internal standard. Infrared absorption spectrum was measured with FT/IR-300E of JASCO Ltd. Mass spectrometry was conducted with GC-Mate of JOEL, Ltd. by a direct method. A melting point was measured with a melting point measuring unit of Yanagimoto.

### Example 1

### <Synthesis of o-phenylenebis(N,N'-dimethyl-N,N'-ethylene)guanidine (BG)>

A dichloromethane (30 mL) solution of 2-chloro-1,3-dimethylimidazolium (95%, 18.9 g, 108 mmol) was slowly added to a dichloromethane (100 mL) solution of o-phenylenediamine (5.28 g, 48.7 mmol) and triethylamine (30.6 mL, 219.8 mmol) under ice cooling. The reaction mixture was stirred at 0°C for 1 hour, and then extracted with a 10% HCl aqueous solution. The aqueous solution was rendered alkaline with a 20% NaOH aqueous solution, and then extracted with toluene. An organic layer was washed with water, and dried (Na₂SO₄). The solvent was then distilled off to obtain a desired product BG (14.5 g, 99%) as viscous yellow oil.

IR (neat) νₘₐₓ: 1650; ¹H NMR δ: 2.64 (s, 12H), 3.21 (s, 8H), 6.74-6.83 (m, 4H); ¹³C NMR δ: 34.2 (Me), 48.1 (CH₂), 120.2 (CH), 122.3 (CH), 141.7 (C), 153.0 (C); EIMS m/z 300 (M⁺).

Preparation of a hexafluorophosphate salt: Ammonium hexafluorophosphate (NH₄PF₆)(52 mg, 0.32 mmol) was slowly added to an aqueous solution (1.0 mL) of BG (100 mg, 0.33 mmol). The mixture was stirred at room temperature for 10 minutes, and then extracted with dichloromethane. After the post-treatment, the resulting residue was recrystallized from ether to obtain a desired product (130 mg, 86%) as colorless prism crystals (mp 137 to 138°C).

IR (nujol) vₘₐₓ: 1640; ¹H NMR δ: 2.81 (s, 12H), 3.61 (s, 8H), 6.92-7.00 (m, 4H); ¹³C NMR δ: 34.4 (Me), 48.6 (CH₂), 122.6 (CH), 123.6 (CH), 135.1 (C), 156.6 (C); Anal. Calcd for C₁₆H₂₅N₅PF₆: C, 42.95; H, 5.82; N 18.79. Found: C, 42.84; H, 5.58; N, 18.54.

### <Optical resolution using L-(-)--benzoyltartaric acid (L-(-)-DBTA·H₂O)>

### (Experimental procedure)

In a reaction vessel, L-(-)-DBTA·H₂O (2.51 g, 6.66 mmol) was dissolved in ethyl acetate (63 mL), and an ethyl acetate (25 mL) solution of BG (1.00, 3.33 mmol) was added dropwise at room temperature. After the reaction mixture was stirred for 3 hours, precipitates separated were collected by filtration, and the filtered product was dried with air to obtain 3.33 g (98.4%) of colorless prism crystals. The colorless prism crystals (1.00 g, 0.98 mmol) were recrystallized from EtOH to obtain 399 mg (38.2%) of colorless prism crystals.

The single crystals (0.50 x 0.20 x 0.10 mm) were subjected to X-ray crystal structure analysis at room temperature using CCD Diffractometer.
Composition formula and molecular weight: C₅₄H₅₈O₁₇N₆ 1063.07
Form: colorless prism crystal
Melting point: 152 to 153°C
IR (KBr) νₘₐₓ(cm⁻¹): 3420 (OH), 1720 (C=O), 1640 (C=N)
¹H NMR (400 MHz, CD₃OD) δ: -0.37 (3H, t, J=7.1 Hz, CH₂), 1.22 (12 H, s, 4x N-CH₃), 2.06 (2H, q, J=7.1 Hz, CH₂), 2.13-2.20 (8H, m, 4x N-CH₂), 4.35 (4H, s, 4x CH), 5.67-5.77 (4H, m, Ar-H), 5.93-5.97 (8H, m, Ar-H), 6.06-6.11 (4H, m, Ar-H), 6.57-6.59 (8H, m, Ar-H) X-ray crystal structure analysis: Bruker SMAT 1000 CCD (MoKα:λ=0.71069 Å), 298K, a colorless prism, 0.50 x 0.20 x 0.10 mm, orthorhombic, chiral space group P 2₁2₁2₁ (#19), a=14.169(2) Å, b=17.124(3) Å, c=22.783(4) Å, V=5527 (1) Å³, Z=4, Dc=1.277 gcm⁻³, R=0.081, Rw=0.087, GOF=1.24

### <Optical resolution using D-(+)--benzoyltartaric acid (D-(+)-DBTA·H₂O)>

### (Experimental procedure)

In a reaction vessel, D-(+)-DBTA·H₂O (2.51 g, 6.66 mmol) was dissolved in ethyl acetate (63 mL), and an ethyl acetate (25 mL) solution of BG (1.00, 3.33 mmol) was added dropwise at room temperature. After the reaction mixture was stirred for 3 hours, precipitates separated were collected by filtration, and the filtered product was dried with air to obtain 3.31 g (97.9%) of colorless prism crystals. The colorless prism crystals (1.00 g, 0.98 mmol) were recrystallized from EtOH to obtain 338 mg (32.3 %) of colorless prism crystals.

The single crystals (0.50 x 0.48 x 0.13 mm) were subjected to X-ray crystal structure analysis at room temperature using CCD Diffractometer.
Composition formula and molecular weight: C₅₄H₅₈O₁₇N₆ 1063.07
Form: colorless prism crystal
Melting point: 152 to 153°C
IR (KBr) νₘₐₓ(cm⁻¹): 3420 (OH), 1720 (C=O), 1640 (C=N) ¹H NMR (400 MHz, CD₃OD) δ: -0.37 (3H, t, J=7.1 Hz, CH₃), 1.22 (12H, s, 4x N-CH₃), 2.06 (2H, q, J=7.1 Hz, CH₂), 2.13-2.20 (8H, m, 4x N-CH₂), 4.35 (4H, s, 4x CH), 5.67-5.77 (4H, m, Ar-H), 5.93-5.97 (8H, m, Ar-H), 6.06-6.11 (4H, m, Ar-H), 6.57-6.59 (8H, m, Ar-H) X-ray crystal structure analysis: Bruker SMAT 1000 CCD (MoKα:λ=0.71069 Å), 298K, a colorless prism, 0.50 x 0.48 x 0.13 mm, orthorhombic, chiral space group P 2₁2₁2₁ (#19), a=14.169(2) Å, b=17.122(3) Å, c=22.798(4) Å, V=5530(1) Å³, Z=4, Dc=1.277 gcm⁻³, R=0.072, Rw=0.148, GOF=1.71

### Example 2

### <Complex formation of (o-phenylenebis(N,N'-diethyl-N,N'-ethylene)guanidine) and ZnCl₂>

According to the following reaction formula, 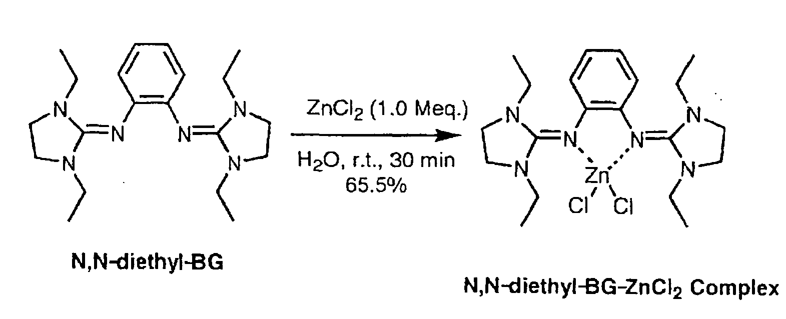 H₂O (0.25 mL) was added to N,N-diethyl BG (51 mg, 0.14 mmol), and ZnCl₂ (23.4 mg, 0.17 mmol, 1.2 Meq.) was added at a time while being stirred. The mixture was stirred for 30 minutes. Colorless crystals precipitated were filtered, dried, and recrystallized from ethyl acetate to obtain 46 mg (65.5%) of colorless prism crystals.

This product was confirmed to have a composition of a desired complex from an elemental analysis value.

Further, X-ray crystal structure analysis of the single crystals was successfully conducted, and it was found that a metal complex was formed.
Composition formula and molecular weight: C₂₀H₃₂Cl₂N₆Zn (Anal. Calcd for: C, 48.74; H, 6.55; N, 17.05.
Found: C, 48.82; H, 6.56; N, 16.89) 492.80
Form: Colorless prism crystal
Melting point: mp 190 to 191°C
IR (KBr) νₘₐₓ(cm⁻¹): 1559 (C=N).
¹H NMR (CDCl₃) δ: 1.14 (12H, t, J=7.1 Hz, CH₂CH₃ x 4), 3.36 (8H, q, J=7.1 Hz, CH₂CH₃ x 4), 3.59 (8H, s, CH₂CH₂ x 2), 6.72-6.74 (2H, m, Ar-H), 6.79-6.81 (2H, m, Ar-H).
¹³C NMR (CDCl₃) δ; 12.1, 42.4, 44.9, 119.7, 120.9, 138.8, 163.0.
FAB-MS m/z: 496 [M(⁶⁸Zn)⁺], 495 [(M(⁶⁶Zn)+1)⁺], 494 [M(⁶⁶ Zn)⁺], 493 [(M(⁶⁴Zn)⁺], 492 [M(⁶⁴Zn)⁺].
X-ray crystal structure analysis: Bruker SMAT 1000 CCD (MoKα: λ=0.71069 Å), 173K, a colorless prism, 0.50 x 0.30 x 0.20 mm, orthorhombic, chiral space group Pna21 (#33), a=12.307(3) Å, b=16.055(4) Å, c=11.840(3) Å, V=2339.5(9) Å³, Z=4,
Dc=1.399 gcm⁻³, R=0.03, Rw=0.033, GOF=0.91.

### Industrial Applicability

According to the invention of this application, a new guanidine compound having a symmetric structure is provided, and this compound can be developed as a new functional substance such as an aid for asymmetric synthesis.

For example, the invention of this application provides a new guanidine compound which is
· an optically resolvable bisguanidine compound,
· a polymer immobilization compound,
· a symmetric chiral benzene base,
· a chiral molecule resolver,
· a recyclable solid-phase chiral identifying agent,
· an organic base or acid substance scavenger,
· a chromatographic carrier for chiral molecule separation or
· a complex with a metal,
and which is useful in the fields of chemical synthesis, separative purification, environmental purification, catalysts and the like.

## Claims

1. An optical resolver containing as an active ingredient a bisguanidine compound represented by the following formula (1) 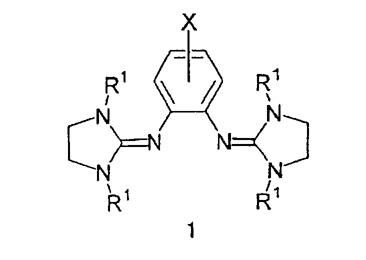 (wherein X represents a hydrogen atom or a substituent, a benzene ring may have the same or different plural substituents bonded thereto, and R¹'s represent the same or different hydrocarbon groups).

2. A chiral molecule separating agent containing as an active ingredient a bisguanidine compound represented by the following formula (1) 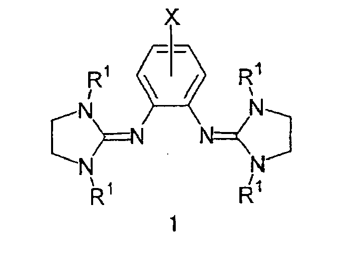 (wherein X represents a hydrogen atom or a substituent, a benzene ring may have the same or different plural substituents bonded thereto, and R¹'s represent the same or different hydrocarbon groups).

3. A complex of a bisguanidine compound represented by the following formula (1) 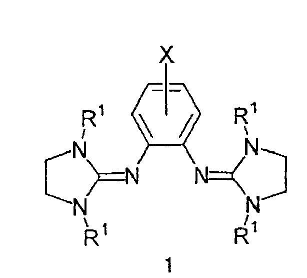 (wherein X represents a hydrogen atom or a substituent, a benzene ring may have the same or different plural substituents bonded thereto, and R¹'s represent the same or different hydrocarbon groups),
and a metal.

4. An organic base or acid substance scavenger containing as an active ingredient a bisguanidine compound represented by the following formula (1) 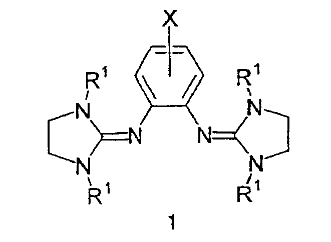 (wherein X represents a hydrogen atom or a substituent, a benzene ring may have the same or different plural substituents bonded thereto, and R¹'s represent the same or different hydrocarbon groups).

5. A process for producing a bisguanidine compound represented by the following formula (1) 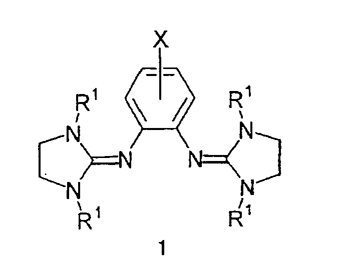 (wherein X represents a hydrogen atom or a substituent, a benzene ring may have the same or different plural substituents bonded thereto, and R¹'s represent the same or different hydrocarbon groups),
which comprises reacting a phenylenediamine compound represented by the following formula 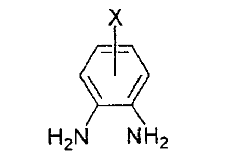 (wherein X represents a hydrogen atom or a substituent, and a benzene ring may have the same or different plural substituents bonded thereto)
with an imidazolinium compound represented by the following formula 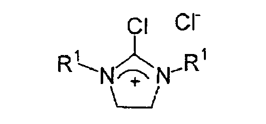 (wherein R¹'s represent the same or different hydrocarbon groups).
